(19) Europäisches Patentamt

European Patent Office

Office européen des brevets

(11) Numéro de publication: **0 202 168**
**B1**

(12) **FASCICULE DE BREVET EUROPEEN**

(45) Date de publication du fascicule du brevet:
01.06.88

(21) Numéro de dépôt: **86401014.5**

(22) Date de dépôt: **13.05.86**

(51) Int. Cl.⁴: **C 07 C 29/78, C 07 C 31/26**

(54) Procédé et installation de cristallisation du mannitol.

(30) Priorité: **15.05.85 FR 8507428**

(43) Date de publication de la demande:
**20.11.86 Bulletin 86/47**

(45) Mention de la délivrance du brevet:
**01.06.88 Bulletin 88/22**

(84) Etats contractants désignés:
**BE DE FR GB IT NL**

(56) Documents cités:
**AU - A - 37 711**
**US - A - 3 632 656**

(73) Titulaire: **Roquette Frères, F-62136 Lestrem (FR)**

(72) Inventeur: **Lemay, Patrick, 29 Rue du Puits Richebourg,
F-62136 Lestrem (FR)**

(74) Mandataire: **Koch, Gustave et al, Cabinet
PLASSERAUD 84, rue d'Amsterdam, F-75009 Paris (FR)**

ACTORUM AG

## Description

L'invention a pour objet un procédé et une installation de cristallisation de mannitol.

Il est connu, notamment par le brevet U.S. N° 3.632.656, de préparer du mannitol cristallisé à partir de solutions aqueuses riches en mannitol et en sorbitol en présence de cristaux de mannitol qui jouent le rôle de germes de cristallisation. La cristallisation est effectuée, selon ce brevet U.S., dans un cristallisoir vertical muni de moyens d'agitation et de régulation de la température propres à maintenir au sein du mélange de sirop et de cristaux une température constante inférieure à la température de saturation du mannitol, la durée de séjour d'une fraction donnée de mélange à l'intérieur du cristallisoir étant de 2 à 15 heures.

Les procédés connus ne donnent pas entièrement satisfaction tant du point de vue de la productivité par unité de volume de l'appareillage que de celui du bilan énergétique.

Or, pour faire face aux contraintes notamment économiques toujours plus sévères, la société titulaire a cherché à mettre au point un procédé et une installation répondant mieux que ceux qui existent déjà aux divers desiderata de la pratique, en particulier précisément du point de vue de la productivité de l'opération de cristallisation par unité de volume de l'appareillage utilisé et du bilan énergétique.

Et elle a trouvé que ce but pouvait être atteint grâce à un procédé de cristallisation en continu du mannitol, caractérisé par le fait qu'un sirop de mannitol et de sorbitol, de préférence exempt de cristaux et de nucléi de mannitol, d'une richesse en mannitol supérieure à 20% et, de préférence, comprise entre 20 et 50%, de préférence entre 30 et 45% et plus préférentiellement encore entre 30 et 42%, d'une teneur en matières sèches de 60 à 90%, de préférence de 60 à 85% et d'une température de 70 à 100°C, est introduit dans une première zone de cristallisation d'axe de préférence sensiblement vertical qu'il est amené à parcourir sous agitation et à l'intérieur de laquelle il est maintenu à une température sensiblement constante, inférieure de 3 à 30°C, de préférence de 3 à 25°C et plus préférentiellement encore de 3 à 20°C à la température de saturation, ce grâce à quoi se produit l'amorçage de la cristallisation qui se traduit par la formation d'un mélange de sirop et de cristaux de mannitol, la durée de séjour moyen d'une fraction donnée de mélange à l'intérieur de cette première zone étant de 10 à 35 heures et de préférence de 15 à 20 heures, de façon telle que ce mélange sortant de cette première zone présente une concentration en cristaux de 5 à 20%, ledit mélange sortant de la première zone étant amené à parcourir, de haut en bas, sous malaxage, une deuxième zone de cristallisation d'axe de préférence sensiblement vertical disposé de préférence sensiblement dans le prolongement de celui de la première zone, un gradient de température globalement décroissant éventuellement modulé de 0,5 à 2°C par heure et de préférence de 1 à 1,5°C étant imposé à l'intérieur de cette deuxième zone au mélange qui la traverse, la température au voisinage de l'extrémité supérieure de la deuxième zone étant de préférence voisine de la température régnant dans la première zone, le mélange qui sort de la deuxième zone se présentant sous la forme d'une masse cristallisée riche en cristaux de mannitol à partir de laquelle on récupère ces derniers, l'amorçage de la cristallisation au niveau de la première zone étant favorisé par le recyclage de préférence au niveau de l'extrémité supérieure de celle-ci d'une fraction du mélange parcourant la seconde zone, fraction qui représente de 10 à 120%, de préférence de 40 à 110% et encore plus préférentiellement de 80 à 100%, de la quantité de sirop introduite dans la première zone, cette fraction, qui est prélevée à un niveau distant des extrémités de cette deuxième zone d'au moins 1/6 de la hauteur totale et de préférence situé dans la partie supérieure de la deuxième zone, notamment entre le 1/6 et la moitié de la hauteur totale, étant avantageusement soumise à la fragmentation des cristaux qu'elle contient, avant son introduction au niveau, de préférence de l'extrémité supérieure, de la première zone.

Pour mettre en œuvre le susdit procédé, on a recours, conformément à l'invention, à une installation comprenant essentiellement deux enceintes d'axes de préférence sensiblement verticaux disposées de préférence l'une au-dessus de l'autre, les axes des deux enceintes étant de préférence sensiblement dans le prolongement l'un de l'autre,

— la première enceinte, ou enceinte d'amorçage de la cristallisation, étant équipée d'une part d'un système d'alimentation en sirop riche en mannitol au voisinage de son extrémité supérieure, d'autre part d'un système d'agitation du contenu de l'enceinte et d'un système de régulation de la température propre à établir à l'intérieur de l'enceinte une température sensiblement constante en tous points et enfin d'un système d'extraction disposé au voisinage de son extrémité inférieure, ce système étant propre à extraire le mélange de sirop et de cristaux formé à l'intérieur de l'enceinte et à acheminer ce mélange à un point situé au voisinage de l'extrémité supérieure de

— la deuxième enceinte, ou enceinte de cristallisation proprement dite et équipée d'un système de malaxage du contenu et d'un système de régulation de la température propre à établir au sein de la masse soumise à cristallisation qui la remplit un gradient de température globalement décroissant de haut en bas, ladite deuxième enceinte étant par ailleurs équipée, au voisinage de son extrémité inférieure, d'un système d'extraction en continu d'un produit fortement enrichi en cristaux de mannitol qui est acheminé par des moyens appropriés vers un système permettant de récupérer les cristaux de mannitol à partir de ce produit,

ladite installation étant en outre équipée d'un système de recyclage vers un point stiué de préférence au voisinage de l'extrémité supérieure de la première enceinte d'une partie du contenu de la deuxième enceinte prélevé à un niveau de la deuxième enceinte correspondant à un point distant de ses extrémités d'au moins 1/6 de la hauteur totale et situé de préférence dans sa partie supérieure, notamment entre le 1/6 et la moitié de la hauteur totale, ledit système de recyclage comprenant avantageusement des moyens de fractionnement des cristaux contenus dans la masse recyclée.

L'invention vise également d'autres dispositions qui s'utilisent de préférence en même temps et dont il sera plus explicitement question ci-après.

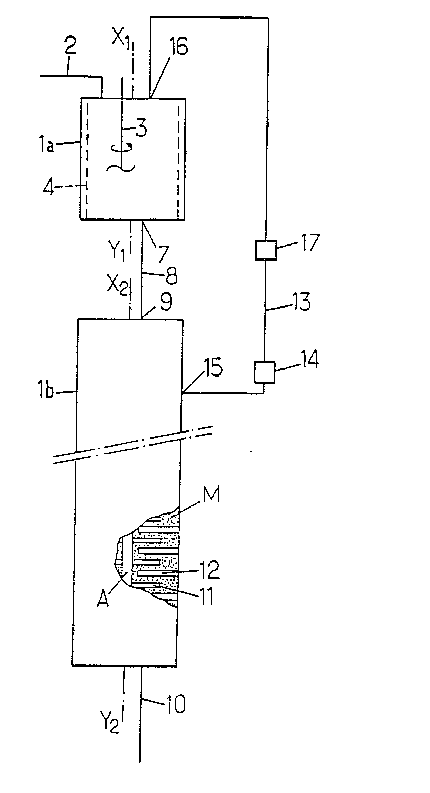

Et elle pourra, de toute façon, être bien comprise à l'aide du complément de description qui suit et du dessin annexé qui sont relatifs à des modes de réalisation avantageux.

La figure unique du dessin montre schématiquement une installation conforme à l'invention.

Se proposant, par conséquent, de préparer du mannitol cristallisé conformément à l'invention, on s'y prend comme suit ou de façon équivalente.

On utilise comme matière première un sirop riche en mannitol, de préférence exempt de cristaux et de nucléi provenant par exemple de l'hydrogénation de sirops riches en fructose; ce sirop présente une teneur en matières sèches d'environ 60 à 90%, de préférence de 60 à 85% en poids, le mannitol entrant pour au moins 20% et, de préférence, de 30 à 45% et plus préférentiellement encore de 35 à 42% en poids sur matières sèches dans sa constitution.

Ce sirop concentré est acheminé vers une première zone de cristallisation d'axe de préférence sensiblement vertical, qu'il est amené à parcourir en continu sous agitation et à l'intérieur de laquelle il est maintenu à une température sensiblement constante, inférieure à la température de saturation, notamment de 3 à 30°C, de préférence de 3 à 25°C et plus préférentiellement encore de 3 à 20°C, ce grâce à quoi se produit l'amorçage de la cristallisation qui se traduit par la formation d'un mélange de sirop et de cristaux de mannitol.

La durée de séjour moyen d'une fraction donnée de mélange à l'intérieur de cette première zone est de 10 à 35 heures et de préférence de 15 à 20 heures, de telle sorte que le mélange sortant de l'enceinte présente une concentration en cristaux de 5 à 20%.

Le mélange sortant de la première zone est amené ensuite, à traverser de haut en bas, sous malaxage, une deuxième zone de cristallisation d'axe de préférence sensiblement vertical, disposé de préférence dans le prolongement de celui de la première zone.

La température du mélange est maintenue, de préférence, au moment de son introduction dans la deuxième zone de cristallisation, à une valeur proche de celle qui règne à l'intérieur de la première zone.

Un gradient de température globalement décroissant de haut en bas de 0,5 à 2°C/heure et de préférence de 1 à 1,5°C, est imposé au mélange, c'est-à-dire à la masse soumise à la cristallisation qui traverse cette deuxième zone.

Le mélange sortant de la deuxième zone se trouve sous la forme d'une masse cristallisée riche en cristaux de mannitol, à partir de laquelle on récupère ces derniers.

La richesse de cette masse en cristaux de mannitol est de 17 à 45%.

L'ensemble de la masse remplissant la deuxième zone de cristallisation parcourt celle-ci à la façon d'un «piston», terme utilisé dans la technique.

L'amorçage de la cristallisation au niveau de la première zone est favorisé par le recyclage, de préférence au niveau de l'extrémité supérieure de celle-ci, d'une fraction du mélange parcourant la seconde zone, cette fraction recyclée représentant de 10 à 120%, de préférence de 40 à 110% et plus préférentiellement encore de 80 à 100% du sirop exempt de nucléi introduit dans la première zone.

Cette fraction est prélevée à un niveau distant des extrémités de cette deuxième zone d'au moins 1/6 de la hauteur totale et de préférence situé dans la partie supérieure de la deuxième zone, notamment entre le 1/6 et la moitié de la hauteur totale.

La fraction recyclée est de plus soumise, de préférence, à un fractionnement des cristaux qu'elle contient, avant son introduction au niveau de l'extrémité supérieure de la première zone.

Grâce au procédé conforme à l'invention, on extrait en continu, au voisinage de l'extrémité inférieure de la deuxième zone de cristallisation, une masse riche en cristaux de mannitol sans qu'il se produise de dérèglement des paramètres du processus de cristallisation, dérèglement qui se répercuterait au niveau de l'étape suivante de séparation de la phase liquide et des cristaux et qui pourrait nécessiter des arrêts intermittents de l'installation. En d'autres termes, ce procédé permet d'arriver à une productivité très favorable par unité de volume de l'appareillage utilisé pour la mise en œuvre du procédé.

Cette productivité est supérieure à celle obtenue dans les procédés de l'art antérieur.

Le débit d'alimentation en sirop riche en mannitol est choisi de façon telle que le temps de séjour moyen d'une fraction donnée de la masse soumise à cristallisation à l'intérieur de la deuxième zone de cristallisation est de 25 à 90 heures, de préférence de 30 à 50 heures; la valeur adoptée dépend des capacités d'échange thermique des moyens comportés par cette deuxième zone et à l'aide desquels est établi, à l'intérieur de ladite zone au sein de la masse soumise à la cristallisation, le gradient de température décroissant dont il a été question plus haut.

La viscosité de la masse soumise à cristallisation qui augmente au fur et à mesure que croît la proportion de cristaux de mannitol, c'est-à-dire dans le sens descendant, fait que la zone de cristallisation est, de préférence, équipée de moyens de refoulement ou d'aspiration propres à faciliter le cheminement de la masse à l'intérieur de la zone.

Par ailleurs, les moyens de malaxage et d'homogénéisation comportés par la deuxième zone de cristallisation doivent être agencés de telle sorte que les zones mortes soient évitées et que l'échange thermique entre la masse soumise à cristallisation et les moyens de refroidissement soit le plus efficace possible.

Le produit extrait de la deuxième zone de cristallisation et qui constitue, comme déjà indiqué, une masse riche en cristaux de mannitol, comprend des cristaux de mannitol d'un spectre granulométrique caractérisé par une faible proportion de fins et de gros cristaux et donc par une forte proportion de cristaux de taille intermédiaire, ce spectre ne variant pas dans le temps, ce grâce à quoi l'étape de traitement suivante, qui consiste à séparer ces cristaux de la phase liquide dans laquelle ils baignent, ne connaît pas de perturbation.

Cette séparation comprend un turbinage et éventuellement un lavage grâce auxquels on récupère la majeure partie de la phase liquide; celle-ci forme des eaux-mères dont la concentration en mannitol est inférieure à celle du sirop riche en mannitol de départ — cette concentration atteint généralement de 7 à 15% — et dans lesquelles on retrouve la presque totalité du sorbitol et des impuretés contenues dans ledit sirop de départ.

Selon la nature cristallographique des cristaux de mannitol, du type α, β ou δ utilisés à titre d'amorçage au niveau de la première zone, on obtient en fin d'opération un mannitol cristallisé de même nature cristallographique, soit respectivement du type α, β ou δ.

Il est possible de faire en sorte que le gradient de température globalement décroissant, imposé à la masse soumise à cristallisation qui traverse la seconde zone, soit modulé, c'est-à-dire se compose par exemple d'une première partie dans laquelle il est effectivement décroissant et d'une seconde partie dans laquelle il est constant, la partie de la deuxième zone correspondant à cette seconde partie du gradient constituant alors en fait une zone de mûrissement.

Cela étant, pour mettre en œuvre le procédé conforme à l'invention, on peut avoir recours à l'installation dont il va être question à présent.

Cette installation comprend essentiellement deux enceintes 1a et 1b avantageusement disposées l'une (1a) au-dessus de l'autre (1b); ces enceintes ont avantageusement la forme de cylindres de révolution d'axes $X_1$, $Y_1$ et $X_2$, $Y_2$ de préférence sensiblement verticaux et de préférence situés dans le prolongement l'un de l'autre.

L'enceinte 1a est équipée:
— d'un système d'alimentation en sirop riche en mannitol au niveau de son extrémité supérieure et représenté schématiquement par une canalisation 2,
— d'un système d'agitation 3, et
— d'un système de régulation de la température schématiquement représenté en 4 et propre à établir une température sensiblement constante en tous points à l'intérieur de l'enceinte.

Le mélange constitué de sirop de mannitol et de cristaux de mannitol, qui s'est formé à l'intérieur de l'enceinte 1a, s'écoule de celle-ci en un point 7 situé au voisinage de l'extrémité inférieure de cette enceinte; à cet endroit l'enceinte peut comporter une canalisation 8 par laquelle le mélange est acheminé vers l'enceinte 1b; on peut également prévoir que l'orifice de sortie de l'enceinte 1a soit disposé en regard de l'orifice d'entrée de l'enceinte 1b, les deux enceintes étant alors juxtaposées.

En règle générale, c'est toutefois la disposition représentée sur la figure qui est adoptée, les deux enceintes étant disposées l'une sous l'autre de préférence dans le prolongement l'une de l'autre, la canalisation 8 jouant simultanément le rôle de canalisation d'extraction pour l'enceinte 1a et de canalisation d'alimentation en mélange de sirop de mannitol et de cristaux de mannitol pour l'enceinte 1b en un point 9 de celle-ci voisin de son extrémité supérieure.

L'enceinte 1b est équipée:
— d'un système de malaxage et de régulation de la température dont il va être question, et
— d'un système d'extraction en continu au niveau de l'extrémité inférieure de l'enceinte et schématiquement représenté par une canalisation 10, ce système étant propre à récupérer la masse riche en cristaux de mannitol obtenue à la sortie de l'enceinte 1b.

Le système de malaxage et de régulation de la température dont il est question ci-dessus peut avantageusement comporter:
— un ensemble de bras de malaxage 11 portés à intervalles réguliers par un arbre rotatif A dont l'axe est confondu avec l'axe $X_2Y_2$ de l'enceinte 1b,
— des nappes de refroidissement 12 disposées en alternance avec les bras malaxeurs 11 et portées par la paroi de l'enceinte 1b, ces nappes de refroidissement étant parcourues par un fluide de refroidissement.

Le système de régulation de la température est agencé de telle sorte qu'il permette d'établir à l'intérieur de l'enceinte 1b un gradient de température globalement décroissant vers le bas.

L'enceinte 1b comporte en outre des moyens globalement représentés par une canalisation 13 comportant une pompe 14 et propres
— à prélever à un niveau 15 de l'enceinte, distant des extrémités de cette deuxième enceinte d'au moins 1/6 de sa hauteur totale et situé de préférence dans la partie supérieure de la deuxième enceinte, notamment entre le 1/6 et la moitié de sa hauteur totale, une fraction de la masse M soumise à cristallisation et parcourant l'enceinte 1b, et
— à recycler cette fraction à un niveau 16 situé au voisinage de l'extrémité supérieure de l'enceinte 1a.

De préférence, toujours, la canalisation 13 comporte des moyens de fragmentation 17, par exemple un broyeur, propres à désagréger les plus gros cristaux et d'éventuels agrégats de cristaux de mannitol contenus dans la fraction recyclée.

La capacité d'échange thermique du système de régulation de température, la vitesse de rotation des moyens de malaxage et la vitesse avec laquelle, sous l'influence des moyens d'aspiration non représentés, la masse soumise à cristallisation parcourt l'enceinte, en d'autres termes, la durée moyenne de séjour d'une fraction donnée de cette masse à l'intérieur de l'enceinte, sont choisies de façon telle que s'établisse, au sein de l'ensemble de la masse soumise à cristallisation, le susdit gradient de température.

On signale que, dans la pratique, le fluide de refroidissement est de l'eau et que l'écart moyen de température en un point donné de l'enceinte, entre cette eau et la masse soumise à cristallisation, est de l'ordre de 2 à 15°C.

*Exemple :*

a) On a recours à une installation conforme à l'invention comportant deux enceintes cylindriques 1a et 1b de volumes utiles respectifs de 1,350 et 3,3 m³.

On introduit dans l'enceinte 1a, avec un débit de 90 l par heure, un sirop de mannitol et de sorbitol ayant une teneur en matières sèches de 78% et comprenant 40,5% en poids sur matières sèches de mannitol, les 59,5% restants étant constitués notamment par du sorbitol et des polysaccharides hydrogénés.

La température du sirop à l'entrée de l'enceinte 1a est d'environ 90°C; la température dans l'enceinte étant de 68°C.

Pour démarrer le processus de cristallisation, on introduit, au niveau de la partie supérieure de l'enceinte 1a, une amorce constituée par du mannitol du type β.

La durée de séjour moyen à l'intérieur de l'enceinte 1a d'une fraction donnée du mélange de sirop et de cristaux de mannitol est d'environ 15 heures.

A la sortie de l'enceinte, ce mélange présente une concentration en cristaux de 8% environ par rapport à la matière sèche.

Le mélange sortant de l'enceinte 1a est amené par la canalisation 8 en un point 9 de l'enceinte 1b situé au voisinage de l'extrémité supérieure de celle-ci.

A l'intérieur de l'enceinte 1b, ce mélange est soumis à un gradient de température globalement décroissant de 1,3°C/h; la température de ce gradient est de 68°C et la température inférieure de 20°C.

Au niveau du point 15 situé au tiers de la hauteur totale à partir de l'extrémité supérieure de l'enceinte 1b, on prélève une fraction de la masse soumise à cristallisation qui la parcourt et on recycle cette fraction vers un point de préférence situé au voisinage de l'extrémité supérieure de l'enceinte 1a en 16, après avoir broyé les agrégats de cristaux.

La fraction recyclée correspond à 100% de la quantité de sirop introduite par la canalisation 2.

La masse riche en cristaux de mannitol du type β extraite au niveau de l'extrémité inférieure de l'enceinte 1b par la canalisation 10 se trouve à une température voisine de 20°C et permet de séparer une quantité de cristaux correspondant en poids à 32% du mélange.

La séparation des cristaux de mannitol est effectuée par turbinage, puis les cristaux sont lavés.

La teneur en mannitol des eaux-mères ainsi récupérées est de 12% et s'élève à 18% en matières sèches après lavage des cristaux.

Le rendement de cristallisation qui est donné par la formule:

$$r = \frac{A - H}{100 - H}$$

dans laquelle:

— A, qui représente la richesse en mannitol du sirop d'alimentation, est de 40,5%;

— H, qui représente la richesse des eaux-mères en mannitol, est de 18% après lavage des cristaux, s'établit à 27,5%.

On produit ainsi par jour 625 kg de mannitol du type β, ce qui correspond à une productivité de 189 kg par jour et par m³ de l'enceinte de cristallisation.

De plus, il ne se produit aucune perturbation nécessitant l'arrêt de l'installation qui fonctionne en continu.

Les cristaux recueillis après turbinage et lavage présentent d'excellentes propriétés physiques et chimiques.

Ces cristaux sont d'une pureté au moins égale à 98%.

b) La même installation et les mêmes conditions opérationnelles sont utilisées.

Cependant, à un moment donné, après avoir atteint l'équilibre du système, la fraction recyclée est prélevée à un niveau situé à l'extérieur du domaine conforme à l'invention.

On constate alors rapidement une évolution des paramètres de cristallisation qui se manifeste après quelques heures par une mauvaise séparation au niveau du turbinage et qui finit par nécessiter l'arrêt de l'installation et l'enlèvement de la masse qui la remplit avant de la remettre en marche sous les conditions conformes à l'invention.

Comme il va de soi et comme il résulte d'ailleurs déjà de ce qui précède, l'invention ne se limite nullement à ceux de ses modes d'application et de réalisation qui ont été plus particulièrement envisagés; elle en embrasse, au contraire, toutes les variantes, notamment celle où l'installation conforme à l'invention comporte une seule enceinte à l'intérieur de laquelle on matérialise, à l'aide de moyens appropriés, les zones d'amorçage de cristallisation, de cristallisation proprement dite et de mûrissement et celle où l'installation conforme à l'invention comporte les enceintes d'amorçage de cristallisation et de cristallisation proprement dites qui ont été décrites dans ce qui précède, la zone de mûrissement étant matérialisée par une troisième enceinte indépendante des deux autres, située de préférence dans leur prolongement et comportant des moyens propres à imposer une température constante à la masse qui la traverse et qui provient de la deuxième enceinte.

**Revendications**

1. Procédé de cristallisation en continu du mannitol, caractérisé par le fait qu'un sirop de mannitol et de sorbitol, d'une richesse en mannitol supérieure à 20%, d'une concentration en matières sèches de 60 à 90%, de préférence de 60 à 85%, et d'une température de 70 à 100°C, est introduit dans une première zone de cristallisation d'axe de préférence sensiblement vertical qu'il est amené à parcourir sous agitation et à l'intérieur de laquelle il est maintenu à une température sensiblement constante, inférieure de 3 à 30°C, de préférence de 3 à 25°C et plus préférentiellement encore de 3 à 20°C, à la température de saturation, ce grâce à quoi se produit l'amorçage de la cristallisation qui se traduit par la formation d'un mélange de sirop et de cristaux de mannitol, la durée de séjour moyen d'une fraction donnée de mélange à l'intérieur de cette première zone étant de 10 à 35 heures et de préférence de 15 à 20 heures, de façon telle que ce mélange sortant de cette première zone présente une concentration en cristaux de 5 à 20%, ledit mélange sortant de la première zone étant amené à parcourir, de haut en bas, sous malaxage, une deuxième zone de cristallisation d'axe de préférence sensiblement vertical disposé de préférence sensiblement dans le prolongement de celui de la première zone, un gradient de température globalement décroissant éventuellement modulé de 0,5 à 2°C/heure, de préférence de 1 à 1,5°C/heure, étant imposé à l'intérieur de cette deuxième zone au mélange qui la traverse, la température au voisinage de l'extrémité supérieure de la deuxième zone étant de préférence voisine de la température régnant dans la première zone, le mélange qui sort de la deuxième zone se présentant sous la forme d'une masse cristallisée riche en cristaux de mannitol à partir de laquelle on récupère ces derniers, l'amorçage de la cristallisation au niveau de la première zone étant favorisé par le recyclage, de préférence au niveau de l'extrémité supérieure de celle-ci d'une fraction du mélange parcourant la seconde zone, fraction qui représente de 10 à 20%, de préférence de 40 à 110% et plus préférentiellement encore de 80 à 100%, de la quantité de sirop introduite dans la première zone, cette fraction étant prélevée à un niveau distant des extrémités de cette deuxième zone d'au moins 1/6 de la hauteur totale.

2. Procédé selon la revendication 1, caractérisé par le fait que la richesse en mannitol du sirop alimentant la première zone est comprise entre 20 et 50%, de préférence entre 30 et 45% et plus préférentiellement encore entre 35 et 42%.

3. Procédé selon la revendication 1, caractérisé par le fait que la fraction recyclée est prélevée à un niveau situé dans la partie supérieure de la deuxième zone, notamment entre le 1/6 et la moitié de la hauteur totale.

4. Procédé selon l'une des revendications 1 à 3, caractérisé par le fait que la fraction recyclée est soumise à une fragmentation des cristaux qu'elle contient, avant son introduction dans la première zone.

5. Procédé selon l'une des revendications 1 à 4, caractérisé par le fait que le gradient globalement décroissant imposé à la masse soumise à cristallisation traversant la deuxième zone, comprend une première partie dans laquelle il est effectivement décroissant et une seconde partie dans laquelle il est constant, la partie de la zone correspondant à cette seconde partie du gradient constituant alors une zone de mûrissement.

6. Installation pour la cristallisation du mannitol, caractérisée par le fait qu'elle comprend essentiellement deux enceintes d'axes de préférence sensiblement verticaux disposées de préférence l'une au-dessus de l'autre, les axes des deux enceintes étant de préférence sensiblement dans le prolongement l'un de l'autre,

— la première enceinte, ou enceinte d'amorçage de la cristallisation, étant équipée d'une part d'un système d'alimentation en sirop riche en mannitol de préférence au voisinage de son extrémité supérieure, d'autre part d'un système d'agitation du contenu de l'enceinte et d'un système de régulation de la température propre à établir à l'intérieur de l'enceinte une température sensiblement constante en tous points et enfin d'un système d'extraction disposé au voisinage de son extrémité inférieure, ce système étant propre à extraire le mélange de sirop et de cristaux formé à l'intérieur de l'enceinte et à acheminer ce mélange à un point situé au voisinage de l'extrémité supérieure de

— la deuxième enceinte ou enceinte de cristallisation proprement dite et équipée d'un système de malaxage du contenu et d'un système de régulation de la température propre à établir au sein de la masse soumise à cristallisation qui la remplit un gradient de température globalement décroissant de haut en bas, ladite deuxième enceinte étant par ailleurs équipée, au voisinage de son extrémité inférieure, d'un système d'extraction en continu d'un produit fortement enrichi en cristaux de mannitol qui est acheminé par des moyens appropriés vers un système adapté à récupérer les cristaux de mannitol à partir de ce produit,

ladite installation étant en outre équipée d'un système de recyclage vers un point situé de préférence au voisinage de l'extrémité supérieure de la première enceinte d'une partie du contenu de la deuxième enceinte prélevé à un niveau distant des extrémités de cette deuxième enceinte d'au moins 1/6 de sa hauteur totale et de préférence situé dans sa partie supérieure, notamment entre 1/6 et la moitié de la hauteur totale, ce système de recyclage comprenant avantageusement des moyens de fractionnement des cristaux contenus dans la masse recyclée.

7. Installation selon la revendication 6, caractérisée par le fait qu'elle comporte une seule enceinte à l'intérieur de laquelle on matérialise, à l'aide de moyens appropriés, les zones d'amorçage de cristallisation, de cristallisation proprement dite et de mûrissement.

8. Installation selon la revendication 6, caractérisée par le fait qu'elle comporte une troisième enceinte disposée de préférence dans le prolongement des deux autres et constituant une zone de mûrissement, cette enceinte comportant des moyens propres à imposer à la masse qui la traverse et qui provient de la deuxième enceinte un gradient de température constant.

## Patentansprüche

1. Verfahren zur kontinuierlichen Kristallisation von Mannit, dadurch gekennzeichnet, dass man einen Sirup von Mannit und Sorbit, dessen Mannitgehalt mehr als 20% beträgt, mit einer Konzentration an Trockensubstanz von 60 bis 90%, vorzugsweise 60 bis 85%, und mit einer Temperatur von 70 bis 100°C, in eine erste Kristallisationszone mit vorzugsweise im wesentlichen vertikaler Achse einführt, welchen man unter Rühren dieselbe durchlaufen lässt und im Inneren derselben bei einer im wesentlichen konstanten Temperatur hält, die um 3 bis 30°C, vorzugsweise 3 bis 25°C und insbesondere 3 bis 20°C niedriger ist als die Sättigungstemperatur, aufgrund dessen die Einleitung der Kristallisation eintritt, die sich in der Bildung eines Gemisches von Sirup und von Kristallen des Mannits äussert, wobei die durchschnittliche Verweildauer einer gegebenen Fraktion des Gemisches im Inneren dieser ersten Zone 10 bis 35 Stunden und vorzugsweise 15 bis 20 Stunden beträgt, derart, dass dieses diese erste Zone verlassende Gemisch eine Konzentration an Kristallen von 5 bis 20% aufweist, wobei man das die erste Zone verlassende Gemisch von oben nach unten unter Mischen eine zweite Kristallisationszone mit vorzugsweise im wesentlichen vertikaler Achse, die bevorzugt im wesentlichen in Verlängerung derjenigen der ersten Zone angebracht ist, durchlaufen lässt, wobei ein insgesamt abnehmender, gegebenenfalls modulierter Temperaturgradient von 0,5 bis 2°C/Stunde, vorzugsweise 1 bis 1,5°C/Stunde, im Inneren dieser zweiten Zone dem Gemisch auferlegt wird, das durch sie gelangt, wobei die Temperatur in der Nähe des äussersten oberen Endes der zweiten Zone vorzugsweise nahe bei der in der ersten Zone vorherrschenden Temperatur liegt, wobei das Gemisch, das die zweite Zone verlässt, in Form einer an Mannitkristallen reichen, kristallisierten Masse, von der ausgehend man diese letztere gewinnt, vorliegt, wobei die Einleitung der Kristallisation im Bereich der ersten Zone durch Recyclisieren, vorzugsweise im Bereich des oberen äussersten Endes derselben, einer Fraktion des die zweite Zone durchlaufenden Gemisches unterstützt wird, wobei diese Fraktion 10 bis 120%, vorzugsweise 40 bis 110% und insbesondere 80 bis 100% der Menge des in die erste Zone eingeleiteten Sirups beträgt, und wobei diese Fraktion in einem Bereich entnommen wird, der von den äussersten Enden dieser zweiten Zone um zumindest 1/6 der Gesamthöhe entfernt ist.

2. Verfahren gemäss Anspruch 1, dadurch gekennzeichnet, dass der Gehalt an Mannit des in die erste Zone eingeführten Sirups zwischen 20 und 50%, vorzugsweise zwischen 30 und 45% und insbesondere zwischen 35 und 42% beträgt.

3. Verfahren gemäss Anspruch 1, dadurch gekennzeichnet, dass die recyclisierte Fraktion in einem Bereich entnommen wird, der in dem oberen Teil der zweiten Zone, insbesondere zwischen 1/6 und der Hälfte der Gesamthöhe liegt.

4. Verfahren gemäss einem der Ansprüche 1 bis 3, dadurch gekennzeichnet, dass die recyclisierte Fraktion einer Fragmentation bzw. Zerkleinerung der Kristalle, die sie enthält, vor ihrer Einführung in die erste Zone unterzogen wird.

5. Verfahren gemäss einem der Ansprüche 1 bis 4, dadurch gekennzeichnet, dass der insgesamt abnehmende Gradient, der der Kristallisation unterzogenen, die zweite Zone durchlaufenden Masse auferlegt wird, einen ersten Teil, in dem der effektiv abnehmend ist, und einen zweiten Teil, in dem er konstant ist, umfasst, wobei der diesen zweiten Teil des Gradienten entsprechende Teil der Zone dann eine Reifungszone darstellt.

6. Vorrichtung zur Kristallisation von Mannit, dadurch gekennzeichnet, dass sie im wesentlichen zwei Behälter mit vorzugsweise im wesentlichen vertikalen Achsen, die bevorzugt übereinander angebracht sind, umfasst, wobei die Achsen der beiden Behälter vorzugsweise im wesentlichen in Verlängerung zueinander angeordnet sind,
— wobei der erste Behälter oder der Behälter für die Einleitung der Kristallisation versehen ist einesteils mit einem System für die Zuführung von an Mannit reichem Sirup, vorzugsweise in der Nähe seines oberen äussersten Endes, anderenteils einem Rührsystem für den Inhalt des Behälters und einem geeigneten Regulierungssystem für die Temperatur, um im Inneren des Behälters eine an sämtlichen Punkten im wesentlichen konstante Temperatur zu erzielen und schliesslich einem Extraktionssystem, das in der Nähe seines unteren äussersten Endes angebracht ist, wobei dieses System geeignet ist für die Extraktion des im Inneren des Behälters gebildeten Gemisches von Sirup und Kristallen und für die Weiterleitung dieses Gemisches zu einer Stelle in der Nähe des oberen äussersten Endes des
— zweiten Behälters für die eigentliche Kristallisation, der versehen ist mit einem System zum Kneten bzw. Rühren des Inhalts und einem geeigneten System zur Regulierung der Temperatur, um innerhalb der Kristallisation unterzogenen Masse, die ihn füllt, einen insgesamt von oben nach unten abnehmenden Temperaturgradienten zu bewirken, wobei dieser zweite Behälter ferner versehen ist in der Nähe seines unteren äussersten Endes mit einem System für die kontinuierliche Extraktion eines stark an Mannitkristallen angereicherten Produkts, das mit Hilfe geeigneter Mittel einem System für die Gewinnung der Mannitkristalle aus diesem Produkt zugeführt wird, wobei diese Vorrichtung ausserdem versehen ist mit einem System für die Recyclisierung zu einer vorzugsweise in der Nähe des oberen äussersten Endes des ersten Behälters gelegenen Stelle eines Teils des Inhalts des zweiten Behälters, der entnommen wird in einem von den äussersten Enden dieses zweiten Behälters um zumindest 1/6 seiner Gesamthöhe eintfernten Bereich, der sich vorzugsweise in seinem oberen Teil, insbesondere zwischen 1/6 und der Hälfte der Gesamthöhe befindet, wobei dieses Recyclisierungssystem vorteilhaft Mittel für die Fraktionierung der in der recyclisierten Masse enthaltenen Kristalle umfasst.

7. Vorrichtung gemäss Anspruch 6, dadurch gekennzeichnet, dass sie einen einzigen Behälter umfasst, in dessen Inneren man mit Hilfe geeigneter Mittel die Zonen für die Einleitung der Kristallisation, für die eigentliche Kristallisation und für die Reifung realisiert.

8. Vorrichtung gemäss Anspruch 6, dadurch gekennzeichnet, dass sie einen dritten Behälter umfasst, der vorzugsweise in Verlängerung der beiden anderen angeordnet ist und eine Reifungszone darstellt, wobei dieser Behälter geeignete Mittel umfasst, um der Masse, die durch ihn gelangt und die dem zweiten Behälter entstammt, einen konstanten Temperaturgradienten aufzuerlegen.

## Claims

1. Method for the continuous crystallization of mannitol, characterized by the fact that a syrup of mannitol and of sorbitol, of a richness in mannitol higher than 20%, of a concentration of dry matter of 60 to 90%, preferably 60 to 85% and of a temperature of 70 to 100°C, is introduced into a first crystallization zone of axis preferably substantially vertical, that it is led to pass through with stirring and inside which it is maintained at a substantially constant temperature, less by 3 to 30°C, preferably by 3 to 25°C and more preferably by 3 to 20°C, than the saturation temperature, due to which there is produced the starting or initiating of the crystallization which is manifested by the formation of a mixture of syrup and of crystals of mannitol, the average dwell time of a given fraction of mixture inside that first zone being from 10 to 35 hours, preferably from 15 to 20 hours, so that this mixture emerging from the said first zone has a concentration of crystals of 5 to 20%, said mixture emerging from the first zone being led to pass through, from top to bottom, under malaxation, a second crystallization zone of axis preferably substantially vertical, arranged preferably substantially in extension of that of the first zone, a temperature gradient globally decreasing possibly modulated of 0.5 to 2°C/hour, preferably of 1 to 1.5°C/hour, being imposed inside this second zone on the mixture which passes through it, the temperature in the vicinity of the higher end of the second zone being preferably close to the temperature inside the first zone, the mixture which emerges from the second zone being in the form of a crystalline mass rich in mannitol crystals from which the latter are recovered, the starting of the crystallization at the level of the first zone being facilitated by the recycling preferably at the level of the upper end of the latter of a fraction of the mixture passing through the second zone, which fraction represents from 10 to 120%, preferably from 40 to 110% and more preferably from 80 to 100%, of the amount of syrup introduced into the first zone, this fraction being taken up at a level spaced from the ends of the said second zone by at least one sixth of its total height.

2. Method according to Claim 1, characterized by the fact that the richness in mannitol of the syrup feeding the first zone is comprised between 20 and 50%, preferably between 30 and 45% and more preferably between 35 and 42%.

3. Method according to Claim 1, characterized by the fact that the recycled fraction is taken up at a level situated in the upper part of the second zone, especially between 1/6 and the midth of the total height of this second zone.

4. Method according to one of Claims 1 to 3, characterized by the fact that the recycled fraction is subjected to fragmentation of the crystals that it contains, before its introduction into the first zone.

5. Method according to one of Claims 1 to 4, characterized by the fact that the globally decreasing gradient imposed to the mass subject to crystallization which traverses the second zone comprises a first part within which it is actually decreasing, and a second part within which it is constant, the part of the zone which corresponds to that second part of the gradient constituting a ripening zone.

6. Installation for the crystallization of mannitol, characterized by the fact that it comprises essentially two vessels of axes preferably substantially vertical arranged preferably one above the other, the axes of the two containers being preferably substantially in extension of one another,

— the first container, or container for initiating crystallization, being equipped on the one hand with a feed system for syrup rich in mannitol preferably in the vicinity of its upper end, on the other hand with a stirring system for the contents of the vessel and a system for regulating the temperature adapted to establish inside the container a substantially constant temperature at all points and finally with an extraction system arranged in the vinicity of its lower end, this system being adapted to withdraw the mixture of syrup and of crystals formed inside the vessel and to lead this mixture to a point situated in the vinicity of the upper end of

— the second container, or container of crystallization proper and equipped with a malaxation system for the contents and with a system for regulation of temperature adapted to establish within the mass subjected to crystallization which fills it a temperature gradient decreasing globally from top to bottom, said second container being furthermore equipped, in the vicinity of its lower end, with a continuous extraction system for a product highly enriched in mannitol crystals which is led by suitable means to a system permitting recovery of the mannitol crystals from this product, said installation being in addition equipped with a system for recycling to a point situated preferably in the vicinity of the upper end of the first vessel of a portion of the contents of the second vessel taken up at a level of the second vessel corresponding to a point spaced from the ends of the second zone by at least one sixth of its total height and preferably situated in the upper part of the second container, particularly between one sixth and one half of its total height, said system of recycling comprising advantageously means for fractionating the crystals contained in the recycled mass.

7. Installation according to Claim 6, characterized by the fact that it comprises a single vessel within which are materialized by way of appropriate means the zones for starting the crystallization, for the crystallization proper and for ripening.

8. Installation according to Claim 6, characterized by the fact that it comprises a third vessel preferably located in extension of the two other vessels and materializing a ripening zone, the said third vessel comprising means proper to impose to the mass traversing it and which comes from the second vessel a temperature substantially constant at all points.